(19)

Europäisches
Patentamt
European
Patent Office
Office européen
des brevets

(11) EP 4 393 382 A1

(12) **EUROPEAN PATENT APPLICATION**

(43) Date of publication:
**03.07.2024 Bulletin 2024/27**

(21) Application number: **23220084.0**

(22) Date of filing: **22.12.2023**

(51) International Patent Classification (IPC):
**A61B 5/01** *(2006.01)*

(52) Cooperative Patent Classification (CPC):
**A61B 5/6802; A61B 5/01; A61B 5/6843;
A61B 5/6898;** A61B 5/0059; A61B 5/681;
A61B 2560/0209

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.12.2022 US 202263477799 P
14.12.2023 US 202318540392**

(71) Applicant: **Apple Inc.
Cupertino, CA 95014 (US)**

(72) Inventor: **TADELE, Wegene H.
Cupertino, 95014 (US)**

(74) Representative: **Zacco Denmark A/S
Arne Jacobsens Allé 15
2300 Copenhagen S (DK)**

(54) **METHODS FOR ON-WRIST DETECT USING TEMPERATURE GRADIENT**

(57) Temperature sensors and/or optical sensors can be used to determine whether an electronic device is worn or not worn by a user of the electronic device (e.g., on-wrist or off-wrist). In some examples, the temperature sensors can serve as a complement or a replacement to optical sensors when determining on-wrist or off-wrist. In some examples, detecting, via temperature sensors, a temperature difference between opposite sides of the wearable device (e.g., front face and back face) indicates on-wrist.

*FIG. 1E*

## Description

## Field of the Disclosure

This relates generally to temperature and/or optical sensing systems and methods, and more particularly to temperature and/or optical sensing systems capable of detecting whether an electronic device is worn by a user of the electronic device.

## Background of the Disclosure

A number of operations of a wearable device can be activated, deactivated or otherwise modified based on whether the wearable device is being worn by a user. However, detecting whether the wearable device is being worn by the user can be inaccurate and/or power intensive.

## Summary of the Disclosure

This relates to algorithms using temperature sensors and/or optical sensors for detecting whether an electronic device is worn by a user of the electronic device. The operation of the electronic device, such as a wearable device, can be adjusted based on whether the electronic device is worn by the user (e.g., on-wrist, on-wrist state, in a first state corresponding to an on-wrist and worn state) or not worn by the user (e.g., off-wrist, off-wrist state, in a second state corresponding to an off-wrist and not worn state). For example, certain functions can be disabled for power-saving, security, or other purposes if the electronic device is off-wrist. In some examples, certain processes (e.g., physiological measurements such as heart or pulse oximetry, contactless payments, or location applications) are enabled based on determining that the electronic device is worn by the user. Some algorithms include continuously sampling optical sensors (e.g., infrared light-emitting diodes (LEDs) or light emitters and light sensors) to determine on-wrist or off-wrist. However, continuously sampling optical sensors may be power intensive, and thus reduces battery life of portable electronic devices. Further, exclusively using optical sensors to detect on-wrist or off-wrist can be susceptible to false positive errors under some conditions. For example, if a wearable device is placed on a surface with a dielectric constant that corresponds to human skin, the optical sensors may falsely identify an on-wrist state.

To reduce power consumption and false positives, the wearable device can leverage temperature gradient measurements from temperature sensors to determine on-wrist (e.g., indicated by a temperature difference measured between a pair of temperature sensors, optionally disposed to measure temperatures at opposite surfaces of the wearable device) or off-wrist (e.g., indicated by little to no temperature difference measured between a pair of temperature sensors, optionally disposed to measure temperatures at opposite surfaces of the wearable device). Temperature sensors consume less power than optical sensors and can serve as a replacement or a complement to optical sensors. In some examples, a first temperature sensor is located at a first surface (e.g., back crystal surface) that is in contact with a user's wrist when the wearable device is worn by the user, and the first temperature sensor is configured to measure a first temperature at the first surface. In some examples, a second temperature sensor is located at a second surface (e.g., front crystal surface) that is exposed to the ambient environment when the wearable device is worn by the user, and the second temperature sensor is configured to measure a second temperature at the second surface. A difference between the first temperature corresponding to the first temperature sensor and the second temperature corresponding to the second temperature sensor indicates on-wrist. In some examples, an on-wrist state is determined when the difference between the first temperature the second temperature is greater than a threshold, such as 0, 0.0001, 0.001, 0.01, 0.1, or 1°C. In some examples, temperature sensors can be exclusively used to determine on-wrist or off-wrist. In some examples, either temperature sensors or optical sensors can be used to determine on-wrist or off-wrist based on opportunistic use. For example, during isothermal conditions (e.g., when an ambient temperature is equal to a wrist temperature corresponding to the user), optical sensors are used to determine on-wrist or off-wrist. In some examples, during non-isothermal conditions (e.g., when the ambient temperature is not equal to the wrist temperature corresponding to the user), temperature sensors are used to determine on-wrist or off-wrist. In some examples, both temperature sensors and optical sensors can be used to determine on-wrist or off-wrist. For example, temperature sensors can be used to determine on-wrist or off-wrist, and optical sensors can be used to verify on-wrist or off-wrist, or vice versa.

## Brief Description of the Drawings

FIGs. 1A-1E illustrate example systems that can include a touch screen and a temperature sensing system according to examples of the disclosure.

FIG. 2 illustrates a block diagram of an example electronic device and a temperature sensing system according to examples of the disclosure.

FIG. 3 illustrates a process for detecting on-wrist or off-wrist using temperature sensors according to examples of the disclosure.

FIG. 4 illustrates a process for detecting on-wrist or off-wrist using temperature sensors or optical sensors according to examples of the disclosure.

FIG. 5 illustrates a process for detecting and verifying on-wrist or off-wrist using both temperature sensors and optical sensors according to examples of the disclosure.

FIG. 6 illustrates a partial cross section of an example wearable device that includes temperature sensors according to examples of the disclosure.

FIG. 7 illustrates a graph depicting temperatures at a front face and a back face of an example wearable device for various characteristics according to examples of the disclosure.

## Detailed Description

In the following description of examples, reference is made to the accompanying drawings which form a part hereof, and in which it is shown by way of illustration specific examples that can be practiced. It is to be understood that other examples can be used, and structural changes can be made without departing from the scope of the disclosed examples.

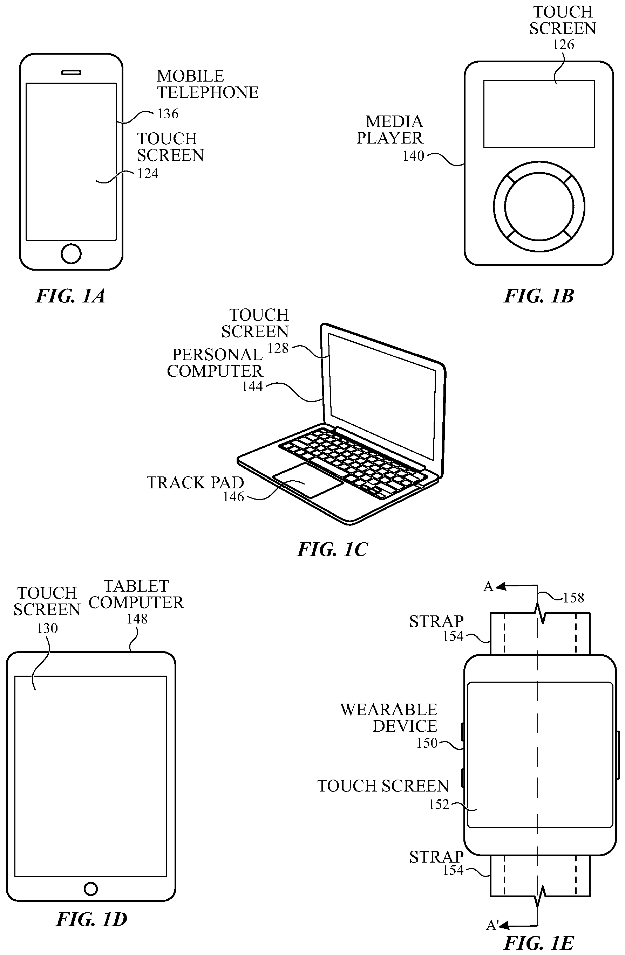

FIGs. 1A-1E illustrate examples of systems, optionally with touch screens (e.g., capacitive touch screens), that can include an example temperature sensing system according to examples of the disclosure. FIG. 1A illustrates an example mobile telephone 136 that optionally includes a touch screen 124 and can include an example temperature sensing system according to examples of the disclosure. FIG. 1B illustrates an example digital media player 140 that optionally includes a touch screen 126 and can include an example temperature sensing system according to examples of the disclosure. FIG. 1C illustrates an example personal computer 144 that optionally includes a touch screen 128 and trackpad 146 according to examples of the disclosure. FIG. 1D illustrates an example tablet computing device 148 that optionally includes a touch screen 130 and can include an example temperature sensing system according to examples of the disclosure. FIG. 1E illustrates an example wearable device 150 (e.g., a watch) that optionally includes a touch screen 152 and can include an example temperature sensing system according to examples of the disclosure. Wearable device 150 can be coupled to a user via strap 154 or any other suitable fastener. It should be understood that the example devices illustrated in FIGs. 1A-1E are provided by way of example, and other devices can include an example temperature sensing system according to examples of the disclosure. Additionally, although the devices illustrated in FIGs. 1A-1E include touch screens, in some examples, the devices may have a non-touch sensitive display or no display.

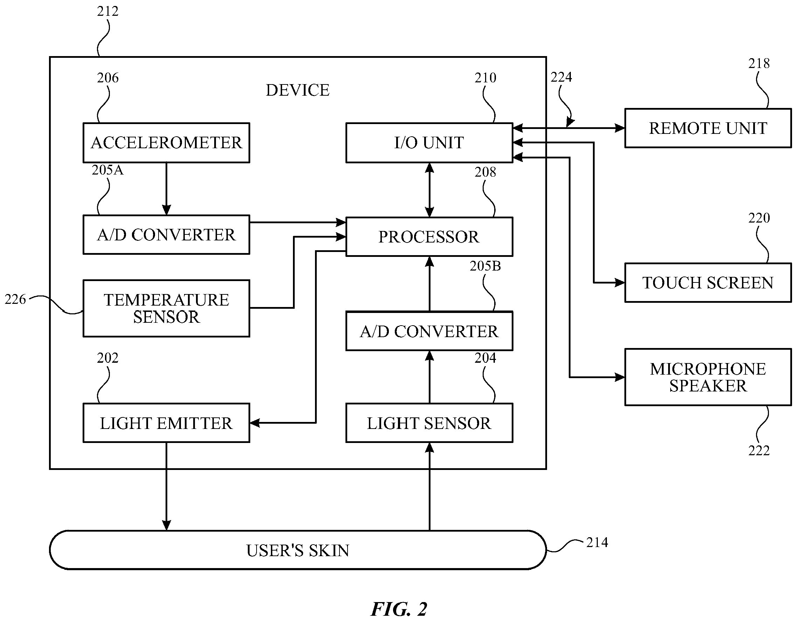

FIG. 2 illustrates a block diagram of an example system implementing a wrist-detection algorithm according to examples of the disclosure. As illustrated in FIG. 2, the block diagram can include a light emitter 202, light sensor 204, analog-to-digital converters (ADCs) 205a and 205b, accelerometer 206, processor 208, and input/output (I/O) unit 210. Although only a single processor 208 is shown, device 212 can include more than one processor or other processing circuitry. These components can be incorporated within a physical device 212 that can be worn or held by a user so as to secure the device to a user's skin (e.g., a user's wrist) or otherwise attached to an article of clothing worn by the user, with the light emitter 202 and light sensor 204 positioned proximate to a user's skin. Alternately, the device 212 can be entirely or partially incorporated within a smartphone or other portable device such that a user can hold the smart phone in a manner to cause the below-described light beam to be reflected from the user's skin back into a light sensor positioned within the smartphone itself. A portion of the light from light emitter 202 can be absorbed by the skin, vasculature, and/or blood, among other possibilities, and a portion can be reflected back to a light sensor 204 co-located with the light emitter. The signals from the light sensor 204 can include heart rate signals due to the blood pulse wave.

Although illustrated in FIG. 2 as having only a single channel formed by a single light emitter 202 and light sensor 204, in other examples multiple channels can be used in the system. The multiple channels can be created by increasing the number of emitter/sensor pairs, where each emitter/sensor pair can create a new channel, for example. In other examples, multiple channels can be created using different light paths from one emitter to multiple sensors (e.g., one emitter and five sensors can produce five light paths). In yet other examples, multiple channels can be created using different light paths from multiple emitters to multiple sensors (e.g., two emitters and two sensors can produce four light paths including two paths from a first emitter to each of the two sensors and two paths from a second emitter to each of the two sensors). The one or more light emitters can produce light in ranges corresponding to infrared (IR), green, amber, blue and/or red light, among other possibilities. In some examples, a light emitter can be a light emitting diode (LED) and a light sensor can be a photodiode.

The accelerometer 206 can provide acceleration output signals indicative of acceleration due to movements of the device or user. For example, the device 212 can be worn on a user's wrist, and the accelerometer output signals can be indicative of the arm movements (e.g., arm swings, rotations, etc.) made by the user. In some examples, the accelerometer can be a three-axis accelerometer providing three-dimensional acceleration outputs (e.g., three channels of acceleration outputs). Additionally, device 212 can include a gyroscope (not shown) that can provide output signals indicative of the orientation of the device to the processor. The output signals from the accelerometer or gyroscope can also be used as inputs to wrist-detection algorithms.

In operation, the light emitter 202 can transmit a light beam to the user's skin 214, and the light beam can be reflected by the user's skin 214 and received by the light sensor 204. The light sensor 204 can convert this light into an electrical signal indicative of the intensity thereof. This electrical signal can be in analog form and can be converted into digital form by ADC 205b. The digital signal from the ADC 205b can be fed to the processor 208. Alternatively, the digital signal can be stored in a memory or a buffer external to processor 208. The outputs of the accelerometer 206 can also be converted to digital form using ADC 205a and either fed to processor 208 or alternatively stored in a memory or a buffer external to processor 208. The processor 208 can receive the digital signals from the light sensor 204 and the digital signals from the accelerometer 206, and can process these signals to determine whether device 212 is worn by a user ("on-wrist") or not worn by the user ("off-wrist").

As mentioned above, the device 212 can include a wearable device. The wearable device can correspond to a watch, a fitness tracker, or any other device (e.g., optionally used to measure physiological signals associated with the user). The wearable device can attach to the user around the wrist (e.g., corresponding to layer of skin at a user's wrist), head, over the eyes, or on any surface of the body that is suitable for measuring physiological signals (e.g., internal body temperature, heart rate, or pulse oximeter) associated with the user.

The wearable device can include a front face (also referred to herein as a "front surface"), a housing, and a back face (also referred to herein as a "rear face" or a "back surface"). The front face is sometimes referred to herein as the "front crystal" of the wearable device, and the back face is sometimes referred to herein as the "back crystal" of the wearable device. However, it should be understood that the front face and back face generally refer to a front or back substrate such as glass, plastic, resin, gel, or crystal. For example, the front face and the back face can protect internal components of the wearable device, but also allow for optical transmission from a display screen (e.g., touch screen) and/or optical sensors. In some examples, the wearable device includes one or more temperature sensors 226, such as absolute temperature sensors, communicatively coupled to the processor 208. For example, the wearable device can include two temperature sensors disposed on opposite sides of the wearable device (e.g., back face and front face). In some examples, a first temperature sensor can be disposed on a first surface (e.g., back face) of the device 212 that is in contact with a user's wrist when the wearable device is worn by the user, and the first temperature sensor is configured to measure a first temperature at the first surface.

In some examples, the first temperature sensor within the wearable device can be used to measure temperatures associated with an optical system located at the back face. In such examples, the first temperature sensor can be configured to measure the temperature at a location or region inside the wearable device (e.g., optionally closer to the back face). Alternatively, or additionally, the temperature sensing system can be used to measure the temperature outside of the wearable device such as the temperature of air contacting back face, or the temperature of other objects at least partially in contact with, or overlapping, the back face (e.g., skin temperature at the wrist).

In some examples, a second temperature sensor is located at a second surface (e.g., front face) that is exposed to the ambient environment when the wearable device is worn by the user, and the second temperature sensor is configured to measure a second temperature at the second surface. The second temperature sensor within the wearable device can be used to measure temperatures associated with a touch screen located at the front face. In such examples, the second temperature sensor can be configured to measure the temperature at a location or region inside wearable device (e.g., optionally closer to the front face). Alternatively, or additionally, the second temperature sensor can be used to measure the temperature outside of wearable device, such as an ambient temperature or temperature of air contacting the front face, or the temperature of other objects at least partially in contact with, or overlapping the front face.

To determine on-wrist or off-wrist, the processor 208 can compare the temperatures at the front face and the back face of the wearable device. As descried in detail below, a predetermined (fixed or configurable) difference between the first temperature corresponding to the first temperature sensor at the back face and the second temperature corresponding to the second temperature sensor at the front face indicates on-wrist.

The I/O unit 210 can take the form of one or more of a storage device, a visual display, an audible annunciator, a touch screen integrated with device 212, or other output indicator. The I/O unit 210 can, under program control from the processor 208, provide, for example, historical information in visual (e.g., numeric, tabular, graphic) or audible (e.g., synthesized voice or tone) form of a detected heart rate over a period of time. The I/O unit 210 can also provide, under control of the processor 208, average heart rate information or statistical information of the heart rate over a prior time period or periods. As a further example, the I/O unit 210 can provide current heart rate values as "real time" or instantaneous heart rate values displayed to the user periodically (e.g., every second) during the course of an ongoing exercise program.

The I/O unit 210 can be coupled to one or more of remote unit 218, touch screen 220 and microphone/speaker 222 or other device via wired or wireless communication links 224. The remote unit 218 can be a smart phone or other I/O device conveniently carried or worn by the user, or can be a distant computer or data server such as the user's home computer or the user's cloud storage service. The I/O unit 210 can receive input from the remote unit 218 or can receive input from the user by means of the touch screen 220 and/or the microphone/speaker 222. For example, I/O unit 210 can receive notifications from a remote unit 218 (e.g., a smartphone) that can be displayed on touch screen 220 when the device 212 is determined by processor 208 to be on-wrist. I/O unit 210 can also route audio information for phone calls between the remote unit 218 and the microphone/speaker 222 of device 212.

Detecting whether device 212 is worn by a user or not can be useful for optimizing operation of device 212. For example, applications requiring the device 212 be properly secured on a user's wrist can be disabled to save power when the device is determined to be off-wrist. Additionally or alternatively, when the device is off-wrist, the data from a sensor (e.g., light sensor 204) can be assumed to be compromised (e.g., bad or invalid) data when the device is determined to be off-wrist. For example, many health or fitness applications can be disabled when the device 212 is off-wrist, and/or data collected by sensors, such as heart rate data, that can be corrupted when the device is not properly secured to a user's body can be ignored or discarded. Likewise, when the device is off -wrist, one or more components of device 212 can be powered down (e.g., one or more processors can be powered down) for additional power savings. Additionally, when the device 212 is determined to be off-wrist, the device 212 can indicate to a remote unit 218 (e.g., a remote device) not to send notifications or calls to the device 212 (and/or device 212 can be configured to not receive or operate in response to said notifications or calls). Similarly, determining that the device 212 is off-wrist can cause the device 212 to require a password to prevent access to the device 212 as a security feature. It should be noted that detecting an on-wrist state or an off-wrist state is an example of detecting or not detecting whether the fastening of device 212 (e.g., wearable device) to the body. In some examples, detecting on-wrist state or an off-wrist state includes detecting whether or not the wearable device 212 is attached to the user around the head, over the eyes, or on any surface of the body that is suitable for measuring physiological signals (e.g., internal body temperature, heart rate, or pulse oximeter) associated with the user.

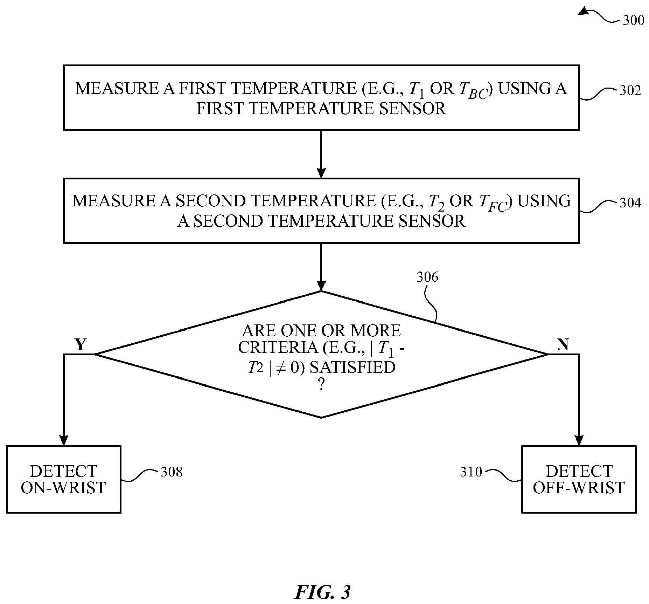

FIG. 3 illustrates an example process 300 for detecting on-wrist or off-wrist using temperature sensors according to examples of the disclosure. As mentioned above, in some examples, temperature sensors consume less power than optical sensors. As such, temperature sensors can be sampled continuously and/or at a higher rate than optical sensors without incurring significant power usage relative to the optical sensors. Further, by exclusively using temperature sensors, detecting on-wrist or off-wrist can be decoupled from other optical (e.g., infrared) sensor applications (e.g., heart rate or pulse oximetry measurements). Temperature sensors can detect a temperature difference between opposite surfaces of a wearable device (e.g., front face which is in contact with the ambient environment when the wearable device is worn by a user and back face which is in contact with a user's wrist when the wearable device is worn by the user). In turn, the temperature difference can be used to determine an on-wrist or off-wrist state.

At block 302, processing circuitry (e.g., processor 208) of an electronic device (e.g., device 212 or wearable device) can receive a first temperature corresponding to a first surface (e.g., back face) of the wearable device from a first temperature sensor. In some examples, the first temperature is an absolute temperature (e.g., $T_1$) measured by a first absolute temperature sensor at the first surface (e.g., back face). In some examples, the first temperature is a back face temperature (e.g., back crystal temperature ($T_{BC}$)). The back face temperature is calculated using the absolute temperature and resistance at the back face of the wearable device.

At block 304, processing circuitry can receive a second temperature corresponding to a second surface (e.g., front face) of the wearable device from a second temperature sensor. In some examples, the second temperature is an absolute temperature (e.g., $T_2$) measured by a second absolute temperature sensor at the second surface (e.g., front face). In some examples, the second temperature is a front face temperature (e.g., front crystal temperature ($T_{FC}$)). The front face temperature is calculated using the absolute temperature and resistance at the front face of the wearable device.

At block 306, processing circuitry can determine on-wrist or off-wrist based on whether one or more criteria are satisfied. In some examples, the one or more criteria include a criterion that is satisfied when a difference between a first temperature corresponding to the first temperature sensor and a second temperature corresponding to the second temperature sensor is greater than a threshold, such as 0, 0.0001, 0.001, 0.01, 0.1, or 1°C. In some examples, the one or more criteria include a criterion that is satisfied when a difference between the first temperature and the second temperature is greater than a percentage difference threshold, such as 0.1, 1, 5, 10, 20, or 30%. In other words, the criterion is satisfied when a predetermined (fixed or configurable) temperature difference exists between the first temperature (e.g., $T_1$ or $T_{BC}$) and the second temperature (e.g., $T_2$ or $T_{FC}$), and therefore by definition the first temperature is not equal to the second temperature.

At block 308, if the one or more criteria are satisfied, the processing circuitry detects that the device is worn by the user or fastened to the user (e.g., on-wrist). When the one or more criteria are satisfied, the temperature corresponding to the back face (e.g., which is in contact with the user's wrist when a wearable device is worn by the user) and the temperature corresponding to the front face (e.g., which is in contact with the user's wrist when a wearable device is worn by the user) are determined to be not equal, and processing circuitry can determine on-wrist.

At block 310, if the one or more criteria are not satisfied, the processing circuitry detects that the device is worn by the user or fastened to the user (e.g., off-wrist). When the one or more criteria are not satisfied, the temperature corresponding to the back face (e.g., which is in contact with the user's wrist when a wearable device is worn by the user) and the temperature corresponding to the front face (e.g., which is in contact with the user's wrist when a wearable device is worn by the user) are determined to be equal, processing circuitry can determine off-wrist. It is understood that process 300 illustrated in FIG. 3 is not limited to the operation as presented, but can include, fewer, additional, and/or simultaneous operations according to various examples.

In some examples, a front crystal module (FCM) aggressor can be located at a front face of the wearable device. A non-limiting example of the front crystal module (FCM) aggressor includes a Wi-Fi module. The front crystal module (FCM) aggressor causes the temperature at the front face (e.g., which is exposed to the ambient environment when a wearable device is worn by the user) to be generally higher (e.g., warmer) compared to the temperature at the back face (e.g., which is in contact with the user's wrist when a wearable device is worn by the user). In some examples, the front crystal module (FCM) aggressor causes the temperature at the front face can be higher (e.g., warmer) compared to the temperature at the back face but higher (e.g., warmer) compared to without the front crystal module (FCM) aggressor. In some examples, due to the temperature difference between the front face and the back face, processing circuitry may exclusively use temperature sensors to determine on-wrist or off-wrist even when a front crystal module (FCM) aggressor (e.g., Wi-Fi) module is located at a front face of the wearable device. However, in some instances (e.g., during transitions corresponding to the front crystal module (FCM) aggressor activity (e.g., Wi-Fi activity)), the temperature at the front face (e.g., which exposed to the ambient environment when a wearable device is worn by the user) is equal to the temperature at the back face (e.g., which is in contact with the user's wrist when a wearable device is worn by the user) for a brief period of time (e.g., less than 0.001, 0.01, 1, or 10 s). Thus, even during transitions corresponding to the front crystal module (FCM) aggressor activity (e.g., when the temperature at the front face and the temperature at the back face are equal), processing circuitry can determine on-wrist if an additional criterion is satisfied. The additional criterion is satisfied when the temperature at the front face and the temperature at the back face are (e.g., approximately) equal for less than a threshold amount of time (e.g., less than 0.001, 0.01, 1, or 10 s). For example, if the temperature at the front face and the temperature at the back face are (e.g., approximately) equal momentarily or briefly, then the processing circuitry can determine that the momentary lack of a difference in temperature is due to front crystal module (FCM) aggressor activity (e.g., Wi-Fi activity) rather than an isothermal condition (e.g., an ambient temperature is equal to a wrist temperature corresponding to the user).

In some examples, the additional criterion is satisfied when the temperature at the front face and the temperature at the back face are (e.g., approximately) equal for less than a threshold number of measurements (e.g., less than 1, 2, 3, 4, 5, or 10 measurements). For example, if the temperature at the front face and the temperature at the back face are (e.g., approximately) equal for a few instances (e.g., and not equal continuously), then the processing circuitry can determine that the lack of a difference in temperature during those few instances is due to front crystal module (FCM) aggressor activity (e.g., Wi-Fi activity) rather than an isothermal condition (e.g., an ambient temperature is equal to a wrist temperature corresponding to the user). In some examples, processing circuitry may monitor a sequence of measurements of the temperature at the front face and the temperature at the back face (e.g., capturing when the measurements are (approximately) equal, and when they are not equal) to determine on-wrist or off-wrist. For example, the number of consecutive instances when the temperature at the front face and the temperature at the back face are (e.g., approximately) equal can affect whether processing circuitry determines on-wrist or off-wrist.

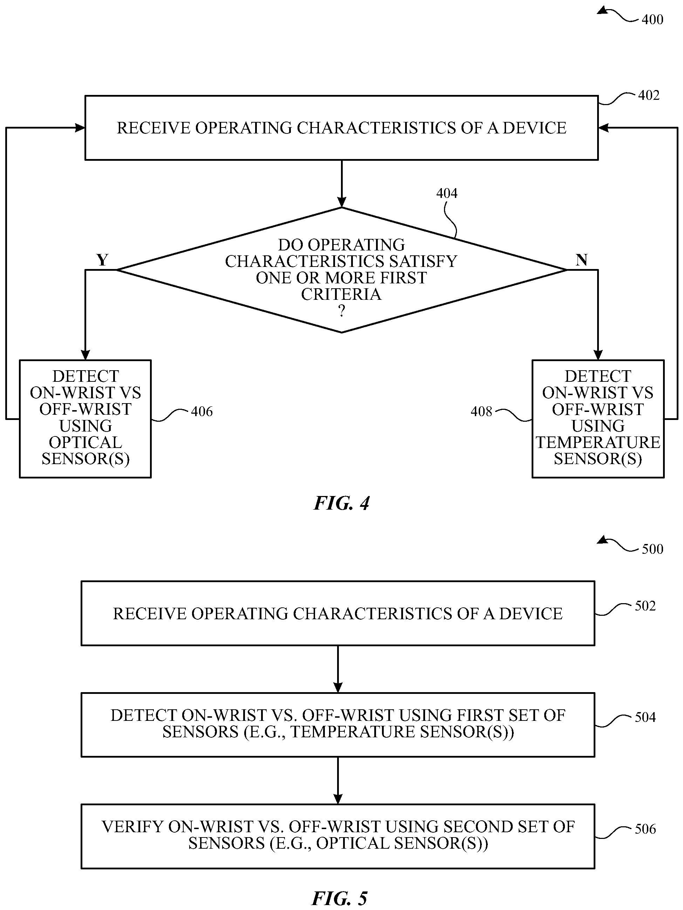

In some examples, either temperature sensors or optical sensors can be used to determine on-wrist or off-wrist based on opportunistic use. Accordingly, FIG. 4 illustrates an example process 400 for detecting on-wrist or off-wrist using temperature sensors or optical sensors according to examples of the disclosure. At block 402, processing circuitry receives characteristics corresponding to the wearable device (e.g., whether isothermal conditions exist, whether the wearable device is configured to measure physiological activity (e.g., heart rate or pulse oximetry), whether a Wi-Fi module is active, and so forth).

At block 404, processing circuitry determines whether the characteristics satisfy one or more criteria. In some examples, the one or more criteria include a criterion that is satisfied when an isothermal condition exists (e.g., when an ambient temperature is equal to a wrist temperature corresponding to the user). In some examples, the one or more criteria include a criterion that is satisfied when the wearable device is configured to measure physiological activity (e.g., heart rate or pulse oximetry). In some examples, the one or more criteria include a criterion that is satisfied when a temperature corresponding to a front face of the electronic device is greater than a threshold temperature (e.g., greater than 35°C).

At block 406, when the above-described one or more criteria are satisfied, processing circuitry uses optical sensors to determine on-wrist or off-wrist. After determining on-wrist or off-wrist, processing circuitry continues to receive updated characteristics corresponding to the wearable device and determines on-wrist or off-wrist for the updated characteristics.

At block 408, when the above-described one or more criteria are not satisfied, processing circuitry uses temperature sensors to determine on-wrist or off-wrist. After determining on-wrist or off-wrist, processing circuitry continues to receive updated characteristics corresponding to the wearable device and determines on-wrist or off-wrist for the updated characteristics.

In some examples, both temperature sensors and optical sensors can be used to determine on-wrist or off-wrist. Accordingly, FIG. 5 illustrates an example process 500 for detecting and verifying on-wrist or off-wrist using both temperature sensors and optical sensors according to examples of the disclosure. At block 502, processing circuitry receives characteristics corresponding to the wearable device (e.g., whether isothermal conditions exist, whether the wearable device is configured to measure physiological activity (e.g., heart rate or pulse oximetry), whether a Wi-Fi module is active, and so forth).

At block 504, processing circuitry determines on-wrist or off-wrist using temperature sensors for the wearable device associated with the above-described characteristics. Alternatively, in some examples, processing circuitry determines on-wrist or off-wrist using optical sensors for the wearable device.

At block 506, processing circuitry determines on-wrist or off-wrist using optical sensors for the wearable device associated with the above-described characteristics to verify the results from temperature sensors. Alternatively, in some examples, processing circuitry determines on-wrist or off-wrist using temperatures sensors for the wearable device verify the results from temperature sensors. It is understood that process 300 illustrated in FIG. 3 is not limited to the operation as presented, but can include, fewer, additional, and/or simultaneous operations according to various examples.

In some examples, processing circuitry determines whether the wearable device is on-wrist or off-wrist using either or both temperature sensors and the optical sensor (e.g., described above at blocks 504 and 506). In some examples, processing circuitry can adjust a sample rate or characteristics of the temperature sensors based on a confidence value associated with using the temperature sensors. For example, if a confidence value associated with using temperature sensors to determine whether the wearable device is on-wrist or off-wrist is greater than a threshold (e.g., greater than a 95, 90, 80, 70, or 50% confidence value), then processing circuitry increases sample rate or other characteristics of the temperature sensors compared to the optical sensors. If the confidence value associated with using temperature sensors to determine whether the wearable device is on-wrist or off-wrist is not greater than the threshold (e.g., not greater than a 95, 90, 80, 70, or 50% confidence value), then processing circuitry decreases sample rate or other characteristics of the temperature sensors compared to the optical sensors. In some examples, processing circuitry can adjust a sample rate or characteristics of the optical sensors based on a confidence value associated with using the optical sensors.

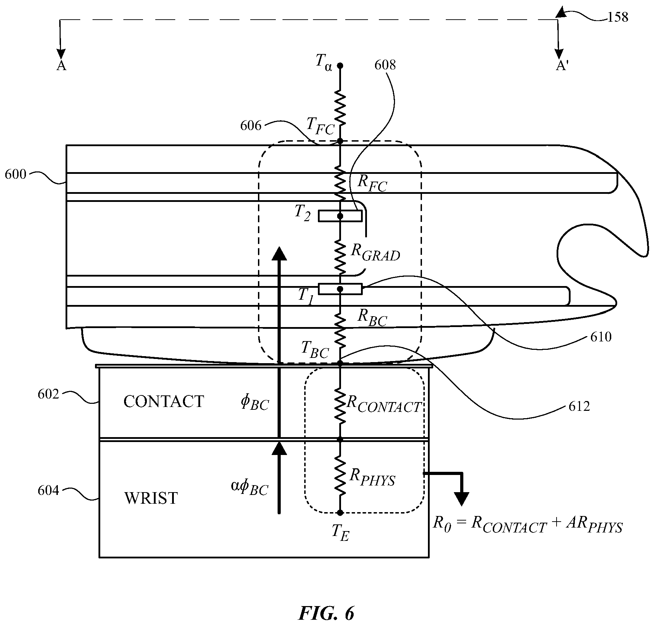

FIG. 6 illustrates a partial cross section of an example wearable device 600 (e.g., cross section 158 corresponding to wearable device 150 in Fig. 1E) that includes temperature sensors 608 and 610 according to examples of the disclosure. The first temperature sensor 610 can measure a first absolute temperature (e.g., $T_1$) at a back face of the wearable device 600, which is in contact (602) with a user's wrist 604 when the wearable device 600 is worn by the user. The second temperature sensor 608 can measure a second absolute temperature (e.g., $T_2$) at a front face of the wearable device 600, which is in contact with ambient environment when the wearable device 600 is worn by the user. In some examples, processing circuitry can determine on-wrist or off-wrist based on a temperature difference between first absolute temperature (e.g., $T_1$) and the second absolute temperature (e.g., $T_2$). In some examples, processing circuitry can determine on-wrist or off-wrist based on a temperature difference between a back crystal temperature 612 (e.g., $T_{BC}$) and a front crystal temperature 606 (e.g., $T_{FC}$). The back crystal temperature 612 (e.g., $T_{BC}$) can be calculated using the following equation: $T_{BC} = T_1 +$

$$\frac{R_{BC}}{R_{Grad}}(T_1 - T_2)$$

. The back crystal temperature 612 (e.g., $T_{BC}$) can be calculated based on the first absolute temperature, a difference between the first absolute temperature and the second absolute temperature, and resistance associated with the back face. The front crystal temperature 606 (e.g., $T_{FC}$) can be calculated using the

$$T_{FC} = T_2 + \frac{R_{FC}}{R_{Grad}}(T_2 - T_1)$$

following equation:                                                 .
The front crystal temperature 606 (e.g., $T_{FC}$) can be calculated based on the second absolute temperature, a difference between the first absolute temperature and the second absolute temperature, and resistance associated with the front face.

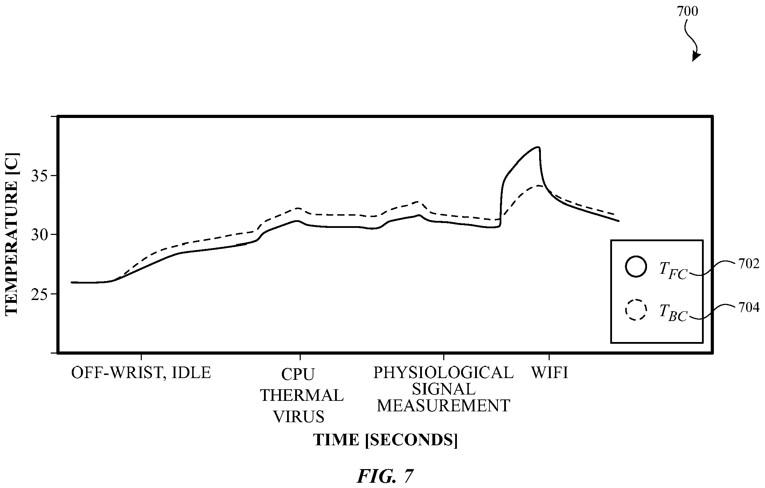

FIG. 7 illustrates a graph 700 depicting temperature at a front face 702 and temperature at back face 704 of an example wearable device for various characteristics according to examples of the disclosure. As illustrated, when the wearable device is off-wrist or idle, the temperature at the front face 702 and the temperature at the back face 704 are (approximately) equal. Processing circuitry can use temperature sensors to determine off-wrist or idle conditions of a wearable device. In some examples, the wearable device includes system-on-chip (SOC) aggressors, such as a CPU thermal virus configured to run any suitable number of processes to increase a temperature within the wearable device. In some examples, the CPU thermal virus can increase the temperature to a predetermined temperature. When the wearable device includes system-on-chip (SOC) aggressors, the temperature at the back face 704 is continuously higher compared to the temperature at the front face 702. In some examples, due to the temperature difference between the front face and the back face, processing circuitry may use temperature sensors to determine on-wrist or off-wrist even when the wearable device includes system-on-chip (SOC) aggressors. In some examples, the wearable device includes back crystal module (BCM) aggressors, such as a physiological signal measurement (e.g., as illustrated), LEDs, or photodiodes at the back face of the wearable device. When the wearable device includes system-on-chip (SOC) aggressors, the temperature at the back face 704 is continuously higher compared to the temperature at the front face 702. In some examples, due to the temperature difference between the front face and the back face, processing circuitry may use temperature sensors to determine on-wrist or off-wrist even when the wearable device includes back crystal module (BCM) aggressors. As mentioned above, in some examples, the wearable device includes front crystal module (FCM) aggressors. In some examples, due to the temperature difference between the front face and the back face, processing circuitry can use temperature sensors to determine on-wrist or off-wrist when a front crystal module (FCM) aggressor (e.g., Wi-Fi) module is located at a front face of the wearable device. However, in some instances (e.g., during transitions corresponding to the front crystal module (FCM) aggressor activity (e.g., Wi-Fi activity)), the temperature at the front face is (e.g., approximately) equal to the temperature at the back face.

Therefore, according to the above, some examples of the disclosure directed to a method. The method can include receiving, via the one or more sensors including a first temperature sensor and a second temperature sensor, sensor data; in accordance with a determination that one or more first criteria are satisfied, the one or more first criteria including a criterion that is satisfied when a difference between a first temperature corresponding to the first temperature sensor and a second temperature corresponding to the second temperature sensor is greater than a threshold, determining that the electronic device is worn by a user of the electronic device; and in accordance with a determination that the one or more first criteria not satisfied, determining that the electronic device is not worn by the user of the electronic device. Additionally or alternatively to one or more of the examples disclosed above, in some examples, the first temperature sensor is disposed at a first surface of the electronic device that is in contact with skin of the user when the electronic device is worn by the user, and the second temperature sensor is disposed at a second surface of the electronic device that is exposed to an ambient environment when the electronic device is worn by the user. Additionally or alternatively to one or more of the examples disclosed above, in some examples, the first surface and second surface are located on opposite sides of the electronic device. Additionally or alternatively to one or more of the examples disclosed above, in some examples, the threshold is 0°C. Additionally or alternatively to one or more of the examples disclosed above, in some examples, the threshold is less than 0.1°C. Additionally or alternatively to one or more of the examples disclosed above, in some examples, the one or more first criteria include a criterion that is satisfied when the first temperature corresponding to the first surface of the electronic device is less than a threshold temperature different than the threshold. Additionally or alternatively to one or more of the examples disclosed above, in some examples, the one or more first criteria include a criterion that is satisfied when the difference between the first temperature corresponding to the first temperature sensor and the second temperature corresponding to the second temperature sensor is not greater than the threshold for less than a threshold number of measurements. Some examples of the disclosure are directed to a method of operating the system described herein or a non-transitory computer readable storage medium storing instructions configured to be executed by one or more processors of the system to cause the processor(s) to perform any of the above operations of the system.

Some examples of the disclosure are directed to an electronic device. The electronic device can comprise a plurality of sensors including one or more temperature sensors and one or more optical sensors. The electronic device can comprise a processor communicatively coupled to the plurality of sensors and configured to in accordance with a determination that one or more first criteria are satisfied, determine whether the electronic device is worn or not worn by a user of the electronic device using the one or more optical sensors and in accordance with a determination that the one or more first criteria are not satisfied, determine whether the electronic device is worn or not worn by the user of the electronic device using the one or more temperature sensors. Additionally or alternatively to one or more of the examples disclosed above, in some examples, the one or more first criteria include a criterion that is satisfied when an ambient temperature is equal to a wrist temperature corresponding to the user. Additionally or alternatively to one or more of the examples disclosed above, in some examples, the one or more first criteria include a criterion that is satisfied when performing physiological measurements using the one or more optical sensors. Additionally or alternatively to one or more of the examples disclosed above, in some examples, the one or more first criteria include a criterion that is satisfied when temperature corresponding to a front crystal surface of the electronic device is greater than a threshold temperature. Additionally or alternatively to one or more of the examples disclosed above, in some examples, the one or more first criteria are not satisfied when a back crystal module aggressor is disposed at a back crystal surface of the electronic device. Some examples of the disclosure are directed to a method of operating the system described herein or a non-transitory computer readable storage medium storing instructions configured to be executed by one or more processors of the system to cause the processor(s) to perform any of the above operations of the system.

Some examples of the disclosure are directed to an electronic device. The electronic device can comprise a plurality of sensors including temperature sensors and optical sensors. The electronic device can comprise a processor communicatively coupled to the plurality of sensors and configured to determine whether the electronic device is worn or not worn by a user of the electronic device using temperature sensors and verify whether the electronic device is worn by the user of the electronic device using optical sensors. Additionally or alternatively to one or more of the examples disclosed above, in some examples, determining whether the electronic device is worn or not worn by the user of the electronic device using the temperature sensors comprises determining whether a difference between a first temperature corresponding to a first temperature sensor of the temperature sensors and a second temperature corresponding to a second temperature sensor of the temperature sensors is greater than a threshold. Additionally or alternatively to one or more of the examples disclosed above, in some examples, wherein the processor is further wherein determining whether the electronic device is worn or not worn by the user of the electronic device using the temperature sensors comprises determining whether the first temperature corresponding to a first surface of the electronic device is less than a threshold temperature. Some examples of the disclosure are directed to a method of operating the system described herein or a non-transitory computer readable storage medium storing instructions configured to be executed by one or more processors of the system to cause the processor(s) to perform any of the above operations of the system.

Some examples of the disclosure are directed to an electronic device. The electronic device can comprise a plurality of sensors including temperature sensors and optical sensors. The electronic device can comprise a processor communicatively coupled to the plurality of sensors and configured to determine whether the electronic device is worn or not worn by a user of the electronic device using at least one of the temperature sensors or the optical sensors; in accordance with a determination that a confidence value associated with using temperature sensors to determine whether the electronic device is worn or not worn by a user of the electronic device is greater than a threshold, decrease one or more characteristics of the optical sensors from a first level to a second level; and in accordance with a determination that the confidence value associated with using the temperature sensors to determine whether the electronic device is worn or not worn by the user is not greater than the threshold, increase the one or more characteristics of the optical sensors from a third level to a fourth level. Additionally or alternatively to one or more of the examples disclosed above, in some examples, the one or more characteristics include a sample rate, an intensity of light, power, or any combination thereof. Additionally or alternatively to one or more of the examples disclosed above, in some examples, the confidence value is reduced when an ambient temperature is equal to a wrist temperature corresponding to the user. Additionally or alternatively to one or more of the examples disclosed above, in some examples, the confidence value is increased when a difference between a first temperature corresponding to a first temperature sensor of the temperature sensors and a second temperature corresponding to a second temperature sensor of the temperature sensors is greater than a threshold temperature difference. Additionally or alternatively to one or more of the examples disclosed above, in some examples, the confidence value is increased when the first temperature corresponding to a first surface of the electronic device is less than a threshold temperature. Some examples of the disclosure are directed to a method of operating the system described herein or a non-transitory computer readable storage medium storing instructions configured to be executed by one or more processors of the system to cause the processor(s) to perform any of the above operations of the system.

Although examples of this disclosure have been fully described with reference to the accompanying drawings, it is to be noted that various changes and modifications will become apparent to those skilled in the art. Such changes and modifications are to be understood as being included within the scope of examples of this disclosure as defined by the appended claims.

Exemplary electronic devices are set out in the following items.

1. An electronic device, comprising:

a plurality of sensors including one or more temperature sensors and one or more optical sensors; and
a processor communicatively coupled to the plurality of sensors and configured to:

in accordance with a determination that one or more first criteria are satisfied, determine, using the one or more optical sensors, whether the electronic device is worn or not worn by a user of the electronic device; and
in accordance with a determination that the one or more first criteria are not satisfied, determine, using the one or more temperature sensors, whether the electronic device is worn or not worn by the user of the electronic device.

2. The electronic device of item 1, wherein the one or more first criteria include a criterion that is satisfied when an ambient temperature is equal to a wrist temperature corresponding to the user.

3. The electronic device of item 1 or 2, wherein the one or more first criteria include a criterion that is satisfied when performing physiological measurements using the one or more optical sensors.

4. The electronic device of any of items 1-3, wherein the one or more first criteria include a criterion that is satisfied when temperature corresponding to a front surface of the electronic device is greater than a threshold temperature.

5. The electronic device of any of items 1-4, wherein the one or more first criteria are not satisfied when a back crystal module aggressor is disposed at a back surface of the electronic device.

6. The electronic device of any of items 1-5, wherein the one or more temperatures sensors includes a first temperature sensor and a second temperature sensor, and wherein determining, using the one or more temperature sensors, whether the electronic device is worn or not worn by the user of the electronic device includes:

receiving, via the one or more temperature sensors including the first temperature sensor and the second temperature sensor, sensor data; in accordance with a determination that one or more second criteria are satisfied, the one or more second criteria including a criterion that is satisfied when a difference between a first temperature corresponding to the first temperature sensor and a second temperature corresponding to the second temperature sensor is greater than a threshold, determining that the electronic device is worn by the user of the electronic device; and in accordance with a determination that the one or more second criteria are not satisfied, determining that the electronic device is not worn by the user of the electronic device.

7. The electronic device of item 6, wherein the first temperature sensor is disposed at a first surface of the electronic device that is in contact with skin of the user when the electronic device is worn by the user, and the second temperature sensor is disposed at a second surface of the electronic device that is exposed to an ambient environment when the electronic device is worn by the user.

8. The electronic device of item 7, wherein the first surface and second surface are located on opposite sides of the electronic device.

9. The electronic device of any of items 7-8, wherein the one or more second criteria include a criterion that is satisfied when the first temperature corresponding to the first surface of the electronic device is less than a threshold temperature.

10. The electronic device of any of items 6-9, wherein the threshold is 0°C.

11. The electronic device of any of items 6-10, wherein the threshold is less than 0.1°C.

12. The electronic device of any of items 6-11, wherein the one or more second criteria include a criterion that is satisfied when a device activity log indicates Wi-Fi activity.

13. The electronic device of any of items 6-12, wherein the one or more second criteria include a criterion that is satisfied when the difference between the first temperature corresponding to the first temperature sensor and the second temperature corresponding to the second temperature sensor is not greater than the threshold for less than a threshold amount of time.

14. The electronic device of any of items 6-13, wherein the one or more second criteria include a criterion that is satisfied when the difference between the first temperature corresponding to the first temperature sensor and the second temperature corresponding to the second temperature sensor is not greater than the threshold for less than a threshold number of measurements.

15. The electronic device of any of items 1-14, wherein the processor is configured to:

in accordance with the determination that the one or more first criteria are satisfied, verify, using the one or more temperature sensors, whether the electronic device is worn or not worn by the user of the electronic device after determining using, the one or more optical sensors, whether the electronic device is worn or not worn by the user of the electronic device; and in accordance with the determination that the one or more first criteria are not satisfied, verify, using the one or more optical sensors, whether the electronic device is worn or not worn by the user of the electronic device after determining, using the one or more temperature sensors, whether the electronic device is worn or not worn by the user of the electronic device.

The foregoing description, for purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the invention and its practical applications, to thereby enable others skilled in the art to best utilize the invention and various embodiments with various modifications as are suited to the particular use contemplated.

**Claims**

1. An electronic device, comprising:

   a plurality of sensors including one or more temperature sensors and one or more optical sensors; and
   a processor communicatively coupled to the plurality of sensors and configured to:

   in accordance with a determination that one or more first criteria are satisfied, determine, using the one or more optical sensors, whether the electronic device is worn or not worn by a user of the electronic device; and
   in accordance with a determination that the one or more first criteria are not satisfied, determine, using the one or more temperature sensors, whether the electronic device is worn or not worn by the user of the electronic device.

2. The electronic device of claim 1, wherein the one or more first criteria include a criterion that is satisfied when an ambient temperature is equal to a wrist temperature corresponding to the user.

3. The electronic device of claim 1 or 2, wherein the one or more first criteria include a criterion that is satisfied when performing physiological measurements using the one or more optical sensors.

4. The electronic device of any of claims 1-3, wherein the one or more first criteria include a criterion that is satisfied when temperature corresponding to a front surface of the electronic device is greater than a threshold temperature.

5. The electronic device of any of claims 1-4, wherein the one or more first criteria are not satisfied when a back crystal module aggressor is disposed at a back surface of the electronic device.

6. The electronic device of any of claims 1-5, wherein the one or more temperatures sensors includes a first temperature sensor and a second temperature sensor, and wherein determining, using the one or more temperature sensors, whether the electronic device is worn or not worn by the user of the electronic device includes:

   receiving, via the one or more temperature sensors including the first temperature sensor and the second temperature sensor, sensor data;
   in accordance with a determination that one or more second criteria are satisfied, the one or more second criteria including a criterion that is satisfied when a difference between a first temperature corresponding to the first temperature sensor and a second temperature corresponding to the second temperature sensor is greater than a threshold, determining that the electronic device is worn by the user of the electronic device; and
   in accordance with a determination that the one or more second criteria are not satisfied, determining that the electronic device is not worn by the user of the electronic device.

7. The electronic device of claim 6, wherein the first temperature sensor is disposed at a first surface of the electronic device that is in contact with skin of the user when the electronic device is worn by the user, and the second temperature sensor is disposed at a second surface of the electronic device that is exposed to an ambient environment when the electronic device is worn by the user.

8. The electronic device of claim 7, wherein the first surface and second surface are located on opposite sides of the electronic device.

9. The electronic device of any of claims 7-8, wherein the one or more second criteria include a criterion that is satisfied when the first temperature corresponding to the first surface of the electronic device is less than a threshold temperature.

10. The electronic device of any of claims 6-9, wherein the threshold is 0°C.

11. The electronic device of any of claims 6-10, wherein the threshold is less than 0.1 °C.

12. The electronic device of any of claims 6-11, wherein the one or more second criteria include a criterion that is satisfied when a device activity log indicates Wi-Fi activity.

13. The electronic device of any of claims 6-12, wherein the one or more second criteria include a criterion that is satisfied when the difference between the first temperature corresponding to the first temperature sensor and the second temperature corresponding to the second temperature sensor is not greater than the threshold for less than a threshold amount of time.

14. The electronic device of any of claims 6-13, wherein the one or more second criteria include a criterion that is satisfied when the difference between the first temperature corresponding to the first temperature sensor and the second temperature corresponding to the second temperature sensor is not greater than the threshold for less than a threshold number of measurements.

**15.** The electronic device of any of claims 1-14, wherein the processor is configured to:

in accordance with the determination that the one or more first criteria are satisfied, verify, using the one or more temperature sensors, whether the electronic device is worn or not worn by the user of the electronic device after determining using, the one or more optical sensors, whether the electronic device is worn or not worn by the user of the electronic device; and
in accordance with the determination that the one or more first criteria are not satisfied, verify, using the one or more optical sensors, whether the electronic device is worn or not worn by the user of the electronic device after determining, using the one or more temperature sensors, whether the electronic device is worn or not worn by the user of the electronic device.

MOBILE
TELEPHONE
136

TOUCH
SCREEN
124

*FIG. 1A*

TOUCH
SCREEN
126

MEDIA
PLAYER
140

*FIG. 1B*

TOUCH
SCREEN
PERSONAL 128
COMPUTER
144

TRACK PAD
146

*FIG. 1C*

TOUCH    TABLET
SCREEN  COMPUTER
130        148

*FIG. 1D*

A

158

STRAP
154

WEARABLE
DEVICE
150

TOUCH SCREEN
152

STRAP
154

A'

*FIG. 1E*

**FIG. 2**

~300

| MEASURE A FIRST TEMPERATURE (E.G., $T_1$ OR $T_{BC}$) USING A FIRST TEMPERATURE SENSOR | ~302 |

| MEASURE A SECOND TEMPERATURE (E.G., $T_2$ OR $T_{FC}$) USING A SECOND TEMPERATURE SENSOR | ~304 |

306

**Y** ← ARE ONE OR MORE CRITERIA (E.G., $| T_1 - T2 | \neq 0$) SATISFIED ? → **N**

| DETECT ON-WRIST | ~308 |

310~ | DETECT OFF-WRIST |

**FIG. 3**

400

402

RECEIVE OPERATING CHARACTERISTICS OF A DEVICE

404

DO OPERATING CHARACTERISTICS SATISFY ONE OR MORE FIRST CRITERIA ?

Y

N

DETECT ON-WRIST VS OFF-WRIST USING OPTICAL SENSOR(S)

406

408

DETECT ON-WRIST VS OFF-WRIST USING TEMPERATURE SENSOR(S)

**FIG. 4**

500

RECEIVE OPERATING CHARACTERISTICS OF A DEVICE

502

DETECT ON-WRIST VS. OFF-WRIST USING FIRST SET OF SENSORS (E.G., TEMPERATURE SENSOR(S))

504

VERIFY ON-WRIST VS. OFF-WRIST USING SECOND SET OF SENSORS (E.G., OPTICAL SENSOR(S))

506

**FIG. 5**

**FIG. 6**

*FIG. 7*

Europäisches
Patentamt
European
Patent Office
Office européen
des brevets

# EUROPEAN SEARCH REPORT

Application Number

EP 23 22 0084

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/162007 A1 (KIM KYUNGMIN [KR] ET AL) 9 June 2016 (2016-06-09) | 1-8,10, 11,13-15 | INV. A61B5/01 |
| A | * paragraphs [0085] - [0088], [0112] * <br> * figures 2A,2B * | 9,12 | |
| X | CN 113 057 602 A (GOERTEK TECH CO LTD) 2 July 2021 (2021-07-02) | 1-8,10, 11,13-15 | |
| A | * machine translation used * <br> * page 6 - page 9 * <br> * figure 1 * | 9,12 | |
| A | US 2021/321953 A1 (PANNEER SELVAM ANJAN [US] ET AL) 21 October 2021 (2021-10-21) <br> * the whole document * | 1-15 | |

**TECHNICAL FIELDS SEARCHED (IPC)**

A61B
G06F

The present search report has been drawn up for all claims

| Place of search | Date of completion of the search | Examiner |
|---|---|---|
| The Hague | 25 March 2024 | Faymann, Juan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 22 0084

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2024

| Patent document cited in search report | | Publication date | Patent family member(s) | | Publication date |
|---|---|---|---|---|---|
| US 2016162007 | A1 | 09-06-2016 | CN | 105700661 A | 22-06-2016 |
| | | | EP | 3032361 A1 | 15-06-2016 |
| | | | KR | 20160069936 A | 17-06-2016 |
| | | | US | 2016162007 A1 | 09-06-2016 |
| CN 113057602 | A | 02-07-2021 | NONE | | |
| US 2021321953 | A1 | 21-10-2021 | US | 2021321953 A1 | 21-10-2021 |
| | | | WO | 2020041715 A1 | 27-02-2020 |

EPO FORM P0459

For more details about this annex : see Official Journal of the European Patent Office, No. 12/82